# EUROPEAN PATENT SPECIFICATION

(11) **EP 0 312 794 B1**
(45) Date of publication and mention of the grant of the patent: **19.05.1993**
(21) Application number: 88115623.6
(22) Date of filing: 22.09.1988
(51) Int. Cl.: C07D 513/14, A61K 31/505, A61K 31/435

(54) **Thiazetidine derivatives**
Thiazetidin-Derivate
Dérivés de thiazétidine

(30) Priority: 22.09.1987 JP 237729/87
(43) Date of publication of application: 26.04.1989
(73) Proprietor: NIPPON SHINYAKU COMPANY, LIMITED, Minami-ku Kyoto-shi Kyoto 601 (JP)
(72) Inventor: Kise, Masahiro, Aneyakoji Nakakyo-ku Kyoto 604 (JP); Ozaki, Masakuni, Joyo-shi Kyoto-Fu 610-01 (JP); Kazuno, Kenji, Ritto-cho Shiga-ken 525 (JP); Tomii, Yoshifumi, Katano-shi Osaka-Fu 576 (JP); Segawa, Jun, 9 Karahashi Biwacho Minami-ku Kyoto 601 (JP); Yasufuki, Shoji, Oeda Nishikyo-ku Kyoto 610-11 (JP)
(74) Representative: Kügele, Bernhard

(56) References cited:
- EP-A- 0 058 392
- CHEMICAL ABSTRACTS, vol. 102, no. 19, 13th May 1985, page 623, no. 166772c, Columbus, Ohio, USA
- CHEMICAL ABSTRACTS, vol. 103, no. 3, 22nd July 1985, page 581, no. 22618e, Columbus, Ohio, USA

## Description

### (Field of the Invention)

The present invention relates to thiazetidine derivatives as represented by the following general formula (I) and salts thereof, which are novel compounds having a bactericidal activity and useful as a remedial medicine for various infectious diseases. 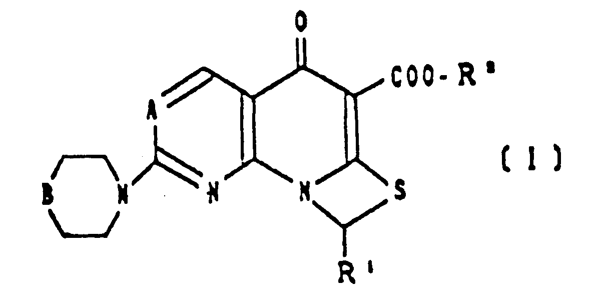
in which R1 represents hydrogen, a linear or branched lower alkyl group having from 1 to 6 carbon atoms or phenyl, 4-fluorophenyl, 2,4-difluorophenyl, 3,4-difluorophenyl or 3,5-difluorophenyl; R2 represents hydrogen or a linear or branched lower all group having from 1 to 6 carbon atoms; A represents N or CX, where X represents a halogen atom; and B represents O or NR³, where R³ represents a hydrogen atom, a linear or branched lower alkyl group having from 1 to 6 carbon atoms, a linear or branched acyl group raving from 1 to 6 carbon atoms, trifluoroacetyl or a linear or branched alkoxycarbonyl group having from 2 to 5 carbon atoms or (5-methyl-2-oxo-1,3-dioxolen-4-yl)methyl.

### (Prior Art)

As synthetic bactericidal agents for remedial medicines to infectious diseases by gram-negative bacteria, nalidixic acid, pyromidic acid, pipemidic acid, enoxacin (AT-2266), ofloxacin (DL-8280) and the like are widely used at present. However, these are not satisfactory for remedy of chronic infectious diseases by Pseudomonas aeruginosa or gram-positive bacterial infectious diseases which are increasing in these days and which are hardly curable diseases. In order to overcome the problem, various kinds of compounds have been synthesized and numerous patent applications have been filed.

The present inventors have heretofore synthesized various kinds of compounds to find out napthyridine compounds and pyridopyrimidine derivatives having an excellent bactericidal activity and have already filed patent applications (JP-A-59-227887 and JP-A-59-210093). The term "JP-A" as used herein means an "unexamined published Japanese patent application"). These patent applications illustrate thiazolonaphthyridine-carboxylic acids and thiazolopyridopyrimidine-carboxylic acid derivatives but do not illustrate the compounds of the present invention. Substituted quinolinecarboxylic acid derivatives having antibacterial actitity are disclosed in European Patent Application 0 058 392, published on August 25, 1982. This patent application also does not illustrate the compounds of the present invention.

### (Problem to be Solved by the Invention)

The known bactericides are limitative with respect to the bactericidal activity and, in addition are not always satisfactory in the point of the safety. The present inventors have repeatedly studied so as to overcome the said problems and have found a group of compounds which have a higher pharmaceutical activity than that of the said known compounds and which are not so toxic and accordingly have achieved the present invention.

Accordingly, the object of the present invention is to provide novel medicines which are far better than the above-mentioned known synthetic bactericides.

### (Means for Solving the Problem)

The compounds of the present invention are novel compounds which are not described in any literatures or publications, and the characteristic feature of the compounds with respect to the chemical structure thereof is that the ring to be formed by the nitrogen atom and the sulfur atom in a 2-mercaptonaphthyridine or 7-mercaptopyridopyrimidine skeleton is a thiazetidine ring.

In the general formula (I), the alkyl group for R¹, R² or R³ is preferably a linear or branched lower alkyl group having from 1 to 6 carbon atoms, which includes, for example, methyl, ethyl, n-propyl, isopropyl, n-butyl, isobutyl, sec-butyl, tert -butyl, n-pentyl, isopentyl, n-hexyl, isohexyl, etc. Examples of aryl represented by R¹ are phenyl, 4-fluorophenyl, 2,4-difluorophenyl, 3,4-difluorophenyl, 3,5-difluorophenyl, etc.

The acyl group for R³ is preferably a linear or branched group having from 1 to 6 carbon atoms, which includes, for example, formyl, acetyl, propionyl, n-butyryl, isobutyryl, valeryl, isovaleryl, pivaloyl, n-hexanoyl, trifluoroacetyl, etc. The alkoxycarbonyl group for R³ is preferably a linear or branched group having frog 2 to 5 carbon atoms; which includes, for example, methoxycarbonyl, ethoxycarbonyl, n-propoxycarbonyl, isopropoxycarbonyl, n-butoxycarbonyl, (5-methyl-2-oxo-1,3-dioxolen-4-yl)methyl, etc.

The halogen for x includes chlorine, bromine, etc.

As examples of salts of the compounds of the formula (I) of the present invention, there may be mentioned, salts of mineral acids such as hydrochloride, sulfate, nitrate, phosphate, hydrofluoride or hydrochloride, salts of organic acids such as formate, acetate, tartarate, lactate, citrate, fumarate, maleate, succinate, methanesulfonate, ethanesulfonate, benzenesulfonate, toluenesulfonate, naphthalenesulfonate or camphorsulfonate, as well as salts of alkali metals or alkaline earth metals such as sodium, potassium or calcium salts.

The compounds of the present invention can be produced, for example, in accordance with the following methods.

### Method-A:

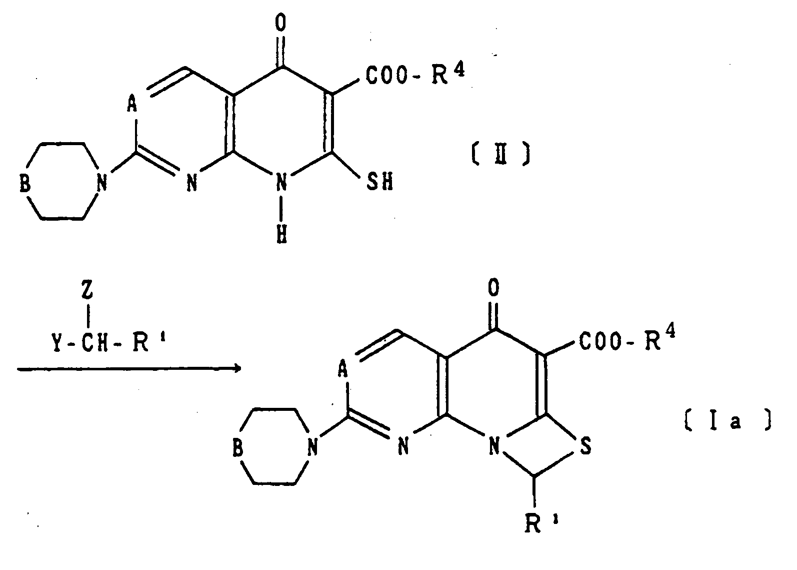

In the reaction formula, R¹, A and B have the same meanings as mentioned above; Y and Z are same or different and each represents a halogen atom; and R⁴ represents an alkyl group.

### Method-B:

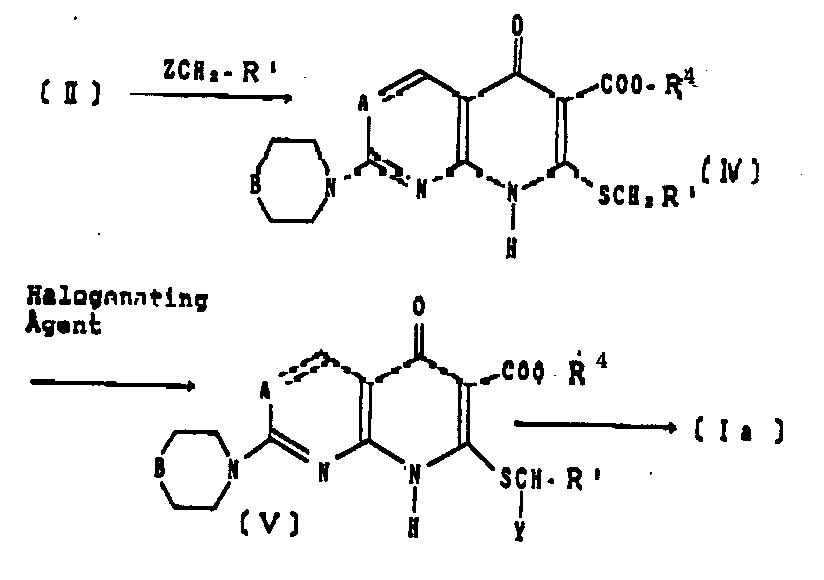

In the reaction formula, R¹, R⁴, A, B, Y and Z have the same meanings as mentioned above.

### Method-C:

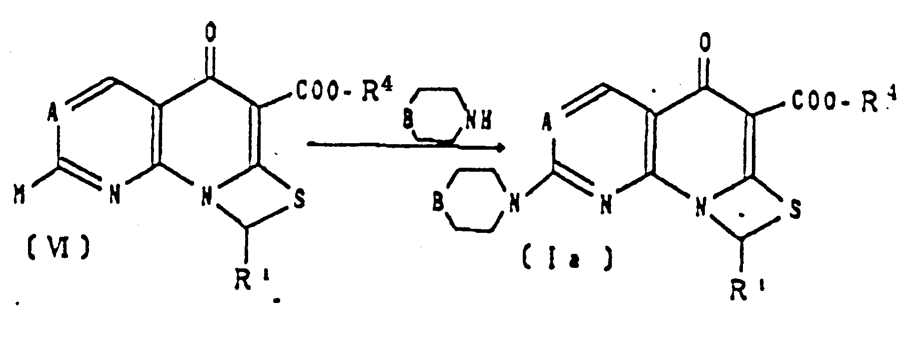

In the reaction formula, R¹, R⁴, A and B have the same meanings as mentioned above; and M represents a halogen atom or an alkylsulfinyl group.

### Method-D:

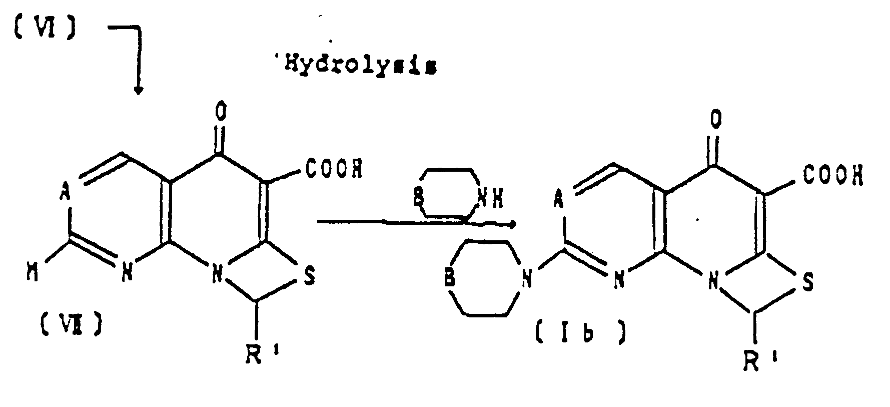

In the reaction formula, R¹, A, B and M have the same meanings as mentioned above.

As is noted from the above-mentioned methods, the compounds of the present invention can be produced by two typical reaction routes. Precisely, one route is a process of forming a thiazetidine ring from a starting material of a naphthyridine-carboxylic acid or pyridopyrimidine-carboxylic acid substituted by a morpholine or a substituted or unsubstituted piperazine (hereinafter referred to as an "amine") (Method-A and Method-B), and the other is a process of forming a thiazetidine ring followed by introduction of an amine into the ring-containing compound (Method-C, Method-D). These processes will be mentioned in detail hereunder.

### Method-A:

The compound (II) is reacted with a dihalide (for example, methylene iodide, ethylidene bromide, benzylidene bromide) in a solvent which is inert to the reaction and in the presence of in deacidificating agent (for example, sodium carbonate, potassium carbonate, triethylamine), generally at 0 to 120°C, so as to be cyclized to prepare the compound (Ia).

As a solvent, for example, an aprotic solvent such as N,N-dimethylformamide, N,N-dimethylacetamide, dimethylsulfoxide or sulforan is preferred. The amount of the dihalide and that of the decarboxylating agent to be used each are preferably from 1.1 to 2.5 mols per mol of the compound (II). In order to accelerate the reaction, from 0.01 to 3.0 molar equivalents of sodium iodide or potassium iodide may be added to the reaction system.

### Method-B:

The compound (II) and a halide (ZCH₂-R¹) are reacted, using the same reaction solvent and deacidificating agent as those in the Method-A, generally at 0 to 80°C to prepare the compound (IV). Subsequently, the resulting compound (IV) is halogenated with a halogonating agent (for example, N-bromosuccinimide, N-chlorosuccinimide) in an inert solvent (for example, halogenated hydrocarbon solvents such as chloroform, dichloromethane or carbon tetrachloride), to prepare the compound (V). The compound (V) is cyclized, using the same reaction solvent and deacidificating agent as those in the Method-A, generally at 0 to 80°C to prepare the compound (Ia).

### Method-C:

The compound (VI) is condensed with an amine to prepare the compound (Ia). In accordance with the process, the compound (VI) is reacted with an amino in a solvent which is inert to the reaction (for example, aprotic solvents such as N,N-dimethylsulfoxide, N,N-dimethylacetamide, dimethylsulfoxide, sulforan, acetonitrile) and optionally in the presence of a deacidificating agent (such as sodium carbonate, potassium carbonate, sodium hydrogencarbonate, potassium hydrogencarbonate or triethylamine), generally at 0 to 80°C, for example at 40 to 60°C. The amount of the amine to be used is from 1.5 to 2.5 mole per mol of the compound (VI).

### Method-D:

The compound (VI) is hydrolyzed with an acid (for example, concentrated sulfuric acid, fuming sulfuric acid, polyphosphoric acid or a mixture thereof) to prepare the compound (VII). The reaction is carried out in the presence of an excess amount (one to 30 times by weight, preferably 5 to 10 times by weight) of in acid as a solvent, generally at 0 to 60°C. The hydrolyzing reaction may also be carried out in 20 to 30 times by weight (preferably 5 to 10 times by weight) of a 1 to 5 % potassium hydroxide or sodium hydroxide-containing aqueous alcohol (e.g., methanol, ethanol, propanol, butanol, preferably tert-butanol), generally at room temperature to 60°C.

Next, the compound (VII) is reacted with an amine in the same solvent as that used in the Method-C to prepare the compound (Ib). The reaction is carried out generally at 0 to 60°C, preferably at 0°C to room temperature.

As still another method, the compounds of the present invention can also be obtained from a compound of a general formula (VIII) shown below, in accordance with the reaction route mentioned below.

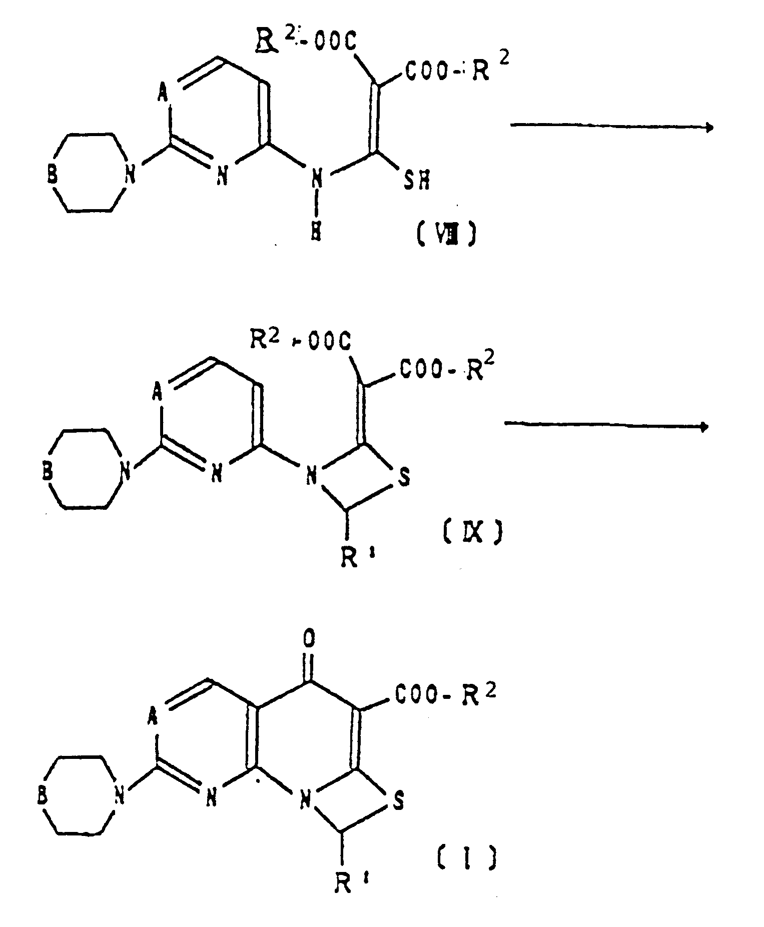

In the reaction formula, R¹, R², A and B have the same meanings as mentioned above.

Precisely, the compound (VIII) is reacted with a dihalide in the presence of a deacificating agent (for example), potassium carbonate) in an inert solvent (for example, N,N-dimethylformamide . The reaction may be carried out substantially in the same manner as the Method-A. Next, the compound (IX) is ring-closed to form the compound (I). The ring-closing reaction can be carried out by a per so known method. For example, there may be mentioned a method of using an acidic substance such as phosphorus oxychloride, phosphorus pentachloride, phosphorus trichloride, thionyl chloride, fuming sulfuric acid, concentrated sulfuric acid, polyphosphoric acid, polyphosphoric acid esters or the like.

In the case of using an acidic substance, the amount of the acidic substance to be used for the process is from one mol to a large excess amount, preferably from 20 to 30 mols, par mol of the compound (IX). The reaction is generally carried out at 0 to 100°C, preferably at 0 to 60°C. In accordance with the kind of the acidic substance used and the reaction condition, the eater moiety of the compound (IX) may be hydrolyzed along with the ring-closure.

When a piperazine is reacted in the above-mentioned reaction steps, one nitrogen of the piperazine compound may optionally be protected with a pertinent protective group (for example, an acyl group) by a known method, if desired, and the final product obtained after reaction may have the protective group, or alternatively, the protective group may be removed after reaction so as to form a final product not containing the protective group.

Further, an N-unsubstituted product may additionally be processed by a per se known method so is to introduce a substituent into the nitrogen atom to give an N-substituted piperazine compound.

For example, known methods may be employed for N-alkylation, which include a method of reaction with a halogenated alkyl, a method of reaction with an alkylsulfuric acid such as dimethylsulfuric acid or a sulfonic acid ester, or a method of reductive alkylating reaction with in aldehyde. The reaction conditions for the respective reactions vary in accordance with the kinds of the raw materials and the alkylating agents used, and the combination of the reaction temperature, the reaction time and the solvent is selected in accordance with the intended products to be obtained.

When (5-methyl-2-oxo-1,3-dioxolen-4-yl)methyl group is to be introduced to the nitrogen atom, the following method, for example, may be applied. 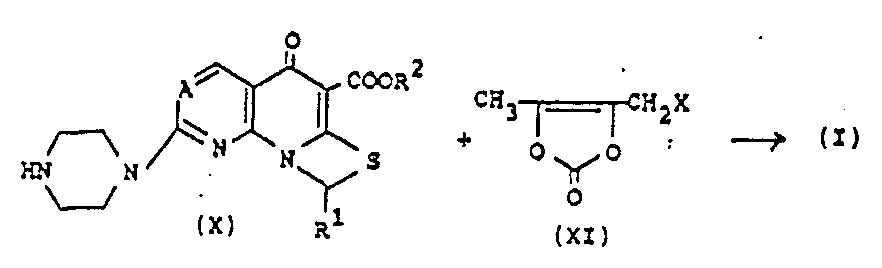
where A, R¹ and R² are the same as those already defined and X is halogen.

Thus, the compounds of the formulae (X) and (XI) are made to react in the absence or presence of an inert solvent in the presence of a base (such as sodium carbonate, potassium carbonate, sodium bicarbonate, potassium bicarbonate, triethylamine, etc.) usually at -20 to 80°C (most preferably at -5°C to ambient temperature) to afford (I).

Preferred solvent used in nonprotonic one such as, for example, N,N-dimethylformamide, dimethyl sulfoxide, and ethers including diglyme.

The amount of (XI) to one mole of (X) is preferably equimole to an excess. The reaction time may vary depending upon the types and amounts of starting materials, solvent and base as well as reaction temperature but, usually, it may be from 2 to 20 hours.

When the compounds obtained by the above-mentioned methods are esters (or that in, R² in the formula is an alkyl group), these may optionally be hydrolyzed, if desired, to convert into the corresponding free carboxylic acids (or that is, R² in the formula is a hydrogen atom). The hydrolyzing reaction is carried out with a large excess amount of an acid (for example, sulfuric acid, fuming sulfuric acid, hydrochloric acid, hydrobromic acid, hydrobromic acid/acetic acid, chlorosulfonic acid, polyphosphoric acid), preferably using the acid in an amount of from 10 to 20 times by weight as a solvent, at room temperature to 110°C. The esters may also be hydrolyzed in 20 to 30 times by weight (preferably 5 to 10 times by weight) of a 1 to 5 % potassium hydroxide or sodium hydroxide-containing aqueous alcohol (e.g., methanol, ethanol, propanol, butanol, preferably tert-butanol) solvent, at room temperature to 60°C with stirring.

Further, the esters may be interesterified, for example, by heating the ester in 10 to 100 times of an alcohol which corresponds to the indended ester to be formed by the interesterification, in the presence of a catalytic amount of a concentrated sulfuric acid, with stirring at 60 to 150°C, preferably at 100 to 110°C.

When the compounds obtained by the invention are carboxylic acids (or that is, R² in the formula is a hydrogen atom), these may optionally be esterified, if desired, to convert into the corresponding esters (or that is, R in the formula is an alkyl group). The esterification reaction can be carried out in accordance with a per se known esterification reaction, for example, by the use of thionyl chloride and an alcohol, an alcohol and a condensing agent (e.g., dicyclohexylcarbodiimide), or an alkyl halide and an alcoholate. In addition, in the case of carboxylic acids, these may be converted into pharmaceutically acceptable salts (for example, sodium salts, potassium salts) by a per se known method, and the salts may also be used like the corresponding free acids.

The starting compounds (II) and (VIII) may be prepared in the same manner as known methods (for example, described in JP-A-59-227887 and JP-A-59-210093).

The starting compound (VI) are new, which may be prepared in accordance with the above-mentioned Method-A and Method-B. Referential Example shown below illustrates the preparation of one of the new compounds (VI).

The final products (I) thus formed may be isolated and purified by per se known means, for example, by concentration, liquid conversion, conversion dissolution, solvent extraction, crystallization, recrystallization, fractional distillation, chromatography or the like.

When the compounds of the present invention are used as a medicine, the compound may be administered to animals inclusive of human, directly or in the form of a medical composition containing the compound, for example, in an amount of from 0.1 % to 99.3 %, preferably 0.5 % to 90 %, in a pharmaceutically acceptable, non-toxic and inert carrier.

As a carrier for the composition, one or more auxiliary substances for medical compositions, for example, solid, semi-solid or liquid diluting agents, fillers and the like can be used. The medical composition is desirably administered in the form of a dose unit. The medical compositions of the present invention can be administered perorally, or by insertion into tissues or local administration (e.g., endermic administration) or perrectal administration. It is a matter of course that the compositions are administered in the form suitable for the said administration routes. In particular, the compositions of the present invention are especially preferably administered perorally.

For use as a medicine for remedy of infectious diseases, it is desired that the composition containing the compound of the present invention is prepared in consideration of the condition of the patient, for example, age or weight, as well an the administration route and the state and condition of the disease, but in general, it is preferred that the composition contains the compound of the present invention in an effective amount of from 50 mg to 1000 mg/day/human adult, more preferably from 100 mg to 300 mg/day/human adult. As the case may be, a smaller amount than the said range may be sufficient, or on the contrary, a larger amount than that may be required. For actual administration, it is desired that the composition is divided into parts for two times or three times administration a day.

### (Example)

The following Referential Example, Examples and Experimental Examples are intended to illustrate the present invention in more detail.

### Referential Example 1:

(1) Ethyl 1-methyl-7-methylthio-4-oxo-4H[1,3]thiazeto[3',2'-1,2]pyrido[2,3-d]pyrimidine-3-carboxylate:
5.58 g of potassium carbonate, 18.95 g of ethylidene bromide and 0.34 g of potassium iodide were added to 160 ml of dry N,N-dimethylformamide (DMF) and heated at 115°C (bath temperature) for 10 minutes. A solution of 6.00 g of ethyl 5-hydroxy-2-methylthio-7-thioxo-7,8-dihydro-pyrido[2,3-d]-pyrimidine-6-carboxylate as dissolved in 400 ml of dry DMF was dropwise added thereto over a period of 1 hour. The whole was continued to be stirred for further 7 hours at the same temperature. The insoluble substances were taken out by filtration and the solvent was taken out by distillation under reduced pressure. The residue was partitioned in chloroform/water, extracted two times with chloroform and washed with an aqueous saturated salt solution. The extract was dried with magnesium sulfate and the solvent was removed by distillation. The residue was recrystallized from ethanol to obtain 4.33 g of the intended compound. m.p. 206 to 207°C.

| Elementary Analysis (C₁₃H₁₃N₂O₃S₂): | | | |
|---|---|---|---|
| Calculated Value (%) | C: 48.28, | H: 4.05, | N: 12.99 |
| Measured Value (%) | C: 48.11, | H: 3.98, | N: 12.93 |

(2) 1-Methyl-7-methylthio-4-oxo-4H[1,3]thiazeto[3',2'-1,2]pyrido[2,3-d]pyrimidine-3-carboxyic acid:
4.10 g of the compound obtained in the above-mentioned stop (1) was dissolved in 50 ml of fuming sulfuric acid and stirred at 60°C (bath temperature) for 60 minutes. The reaction solution was poured onto ice and the precipitate forced was collected by centrifugation. This was washed with water, methanol and ether each three times and dried under reduced pressure to obtain 3.40 g of the intended compound m.p. 225 to 227°C (decomposition).

| Elementary Analysis (C₁₁H₉N₃O₃S₂): | | | |
|---|---|---|---|
| Calculated Value (%) | C: 44.74, | H: 3.07, | N: 14.23 |
| Measured Value (%) | C: 44.74, | H: 2.93, | N: 14.12 |

(3) 1-Methyl-7-methylsulfinyl-4-oxo-4H[1,3]thiazeto[3',2'-1,2]pyrido[2,3-d]pyrimidine-3-carboxylic acid:
1.00 g of the compound obtained in the above-mentioned step (2) was dissolved in 250 ml of chloroform, and 0.77 g of m-chloroperbenzoic acid (m-CPBA) was gradually added thereto. After stirred for 1 hour, the solvent was removed by distillation under reduced pressure. The residue was washed with ethanol and ether and dried under reduced pressure to obtain 0.98 g of the intended compound.
m.p. 208 to 210°C (decomposition).

| Elementary Analysis (C₁₁H₉N₃O₄S₂) | | | |
|---|---|---|---|
| Calculated Value (%) | C: 42.44, | H: 2.91, | N: 13.50 |
| Measured Value (%) | C: 41.83, | H: 3.03, | N: 13.30 |

In the same manner, the following compounds were obtained.
7-Methylsulfinyl-4-oxo-4H[1,3]thiazeto[3',2'-1,2]pyrido[2,3-d]pyrimidine-3-carboxylic acid:
m.p. 300°C or higher.

| Elementary Analysis (C₁₀H₇N₃O₄S₂) | | | |
|---|---|---|---|
| Calculated Value (%) | C: 40.40, | H: 2.37, | N: 14.13 |
| Measured Value (%) | C: 40.06, | H: 2.17, | H: 14.09 |

I R ν ₘₐₓ cm⁻¹: 3000, 1705, 1600, 1580, 1430, 1380, 1340, 1305, 1060, 815

### Example 1:

Ethyl 7-(4-Acetyl-1-piperazinyl)-6-fluoro-4-oxo-4H[1,3]-thiazeto[3,2-a]-1,8-napthyridine-3-carboxylate:
A solution of 3.0 g of ethyl 7-(4-acetyl-1-piperazinyl)6-fluoro-4-hydroxy-2-mercapto-1,8-napthyridine-3-carboxylate as dissolved in 150 ml of chloroform and 50 ml of DMF was dropwise added to a solution obtained by heating and stirring a mixture comprising 4.08 g of methylene chloride, 2.54 g of potassium carbonate and 90 ml of DMF at 60 to 65°C, over a period of 2 hours. The whole was stirred for further 30 minutes at the same temperature and then water was added thereto. This was made weakly acidic with acetic acid and extracted with chloroform. The chloroform layer was separated, washed with water, dried and concentrated. Ethanol was added to the resulting residue, and the crystal formed was taken out by filtration and washed with a small amount of ethanol to obtain 2.87 g of a pale yellow crystal. m.p. 244 to 246°C (decomposition).

### Example 2:

6-Fluoro-7-(1-piperazinyl)-4-oxo-4H[1,3]-thiazeto[3,2-a]-1,8-naphthyridine-3-carboxylic acid hydrochloride:
3.6 g of the compound obtained in Example 1 was suspended in 36 ml of a 5 % aqueous hydrochloric acid solution and heated and stirred at 100°C for 4.5 hours. Alter cooled, the crystal precipitated was taken out by filtration and washed with ethanol and ether in order. The crude crystal thus obtained was recrystallized from ethanol/water (1/1). Thus 2.1 g of a white powdery crystal was obtained. m.p. 235°C (decomposition).

| Elementary Analysis (C₁₄H₁₃FN₄O₃S·HCl): | | | |
|---|---|---|---|
| Calculated Value (%) | C: 45.11, | H: 3.79, | N: 15.03 |
| Measured Value (%) | C: 45.14, | H: 3.84, | N: 14.87 |

### Example 3:

6-Fluoro-7-(4-methyl-1-piperazinyl)-4-oxo-4H[1,3]-thiazeto-[3,2-a]-1,8-napthyridine-3-carboxylic acid hydrochloride:
1.1 g of the compound obtained in Example 2 was suspended in 5 ml of water and neutralized with 2.35 ml of 5 % soduim hydroxide. The crystal precipitated was taken out by filtration. This was washed with water, ethanol and other in order. The crystal was dissolved in 3.6 ml of formic acid, and 1.2 ml of a 37 % aqueous formalin solution was added thereto and heated and stirred for 1.5 hours at 110 to 120°C. After cooled, this was diluted with ethanol and the crystal precipitated was taken out by filtration. The crystal was suspended in a small amount of water and then dissolved with a 5 % aqueous sodium hydroxide solution. The resulting solution was made acidic with a 5 % aqueous hydrochloric acid solution and then diluted with ethanol. The crystal precipitated was taken out by filtration and recrystallized from a mixed solvent of ethanol and water to obtain 0.84 g of a white powdery crystal. m.p. 240°C (decomposition).

| Elementary Analysis (C₁₅H₁₅FN₄O₃S·HCl): | | | |
|---|---|---|---|
| Calculated Value (%) | C: 46.57, | H: 4.17, | N: 14.48 |
| Measured Value (%) | C: 46.49, | H: 4.14, | N: 14.24 |

### Example 4:

(1) Diethyl 3-(5-chloro-6-morpholinylpyridin-2-yl)-[1,3]-thiazetidin-2-ylidenemalonate:
While 4.10 g of methylene iodide, 2.54 g of potassium carbonate and 80 ml of dry DMF were heated and stirred at 50 to 60°C, a solution of 3.18 g of diethyl 5-chloro-6-morpholino-2-pyridinylaminomercaptomethylene-malonate as dissolved in 160 ml of dry DMF was dropwise added thereto over a period of 6 hours. After the addition, the reaction mixture was concentrated under reduced pressure at 50 to 60°C, and the residue was dissolved in ethyl acetate, washed with water, dried and concentrated. n-hexane was added to the residue, and the crystal precipitated was recrystallized from n-hexane/ethyl acetate to obtain 2.01 g of a white crystal. m.p. 142 to 144°C.
(2) 6-Chloro-7-morpholino-4-oxo-4H-[1,3]-thiazeto-[3,2-a]-1,8-naphthyridine-3-carboxylic acid:
While 40 g of fuming sulfuric acid was stirred under ice-cooling, 2.01 g of the compound obtained in the above-mentioned step (1) was gradually added thereto and stirred at room temperature for 12 hours. The reaction solution was added to water, and the colloidal crystal precipitate was taken out by centifugation. The crystal was washed with water and dried to obtain 1.10 g of a pale yellow crystal. m.p. 254°C (decomposition).

| Elementary Analysis (C₁₄H₁₂ClN₃O₄S·H₂O) | | | |
|---|---|---|---|
| Calculated Value (%) | C: 45.23, | H: 3,79, | N: 11.30 |
| Measured Value (%) | C: 45.51, | H: 3.30, | N: 11.29 |

### Example 5:

Ethyl 6-Chloro-1-methyl-7-morpholino-4-oxo-4H[1,3] thiazeto[3,2-a]-1,8-naphthyridine-3-carboxylate:
A solution of 6.10 g of ethyl 6-chloro-4-hydroxy-2-mercapto-7-morpholino-1,8-naphthyridine-3-carboxylate, 6.20 g of ethylidine bromide, 4.56 g of potassium carbonate and 66 mg of potassium iodide as dissolved in 660 ml of dry DMF was blended and heated with an oil bath of 100 to 110°C for 5.9 hours with stirring. After the reaction, the reaction mixture was concentrated at 70°C under reduced pressure. Ice-water was added to the residue, which was then extracted with chloroform. The chloroform layer was washed with water, dried and concentrated, and the residue thus obtained was crystallized with ethyl acetate. The crystal was taken out by filtration and recrystallized from ethyl acetate, to obtain 3.79 g of a pale yellow crystal. m.p. 192 to 193°C.

| Elementary Analysis (C₁₇H₁₈ClN₃O₄S): | | | |
|---|---|---|---|
| Calculated Value (%) | C: 51.58, | H: 4.58, | N: 10.62 |
| Measured Value (%) | C: 51.45, | H: 4.63, | N: 10.62 |

### Example 6:

6-Chloro-1-methyl-7-mopholino-4-oxo-4H[1,3]thiazeto[3,2-a]-1,8-naphthyridine-3-carboxylic acid:
While 76 g of fuming sulfuric acid was stirred under ice-cooling, 3.79 g of the compound obtained in Example 5 was gradually added thereto and stirred at room temperautre. After reacted for 12 hours, the reaction solution was added to ice, and the crystal precipitated was taken out by centrifugation. This was washed with water and dried to obtain 3.62 g or a crude crystal. This was recrystallized from DMF to obtain 2.44 g of a polo yellow crystal. m.p. 242°C (decomposition).

| Elementary Analysis (C₁₅H₁₄ClN₃O₄S): | | | |
|---|---|---|---|
| Calculated Value (%) | C: 48.98, | H: 3.84, | N: 11.42 |
| Measured Value (%) | C: 48.78, | H: 3.82, | N: 11.16 |

### Example 7:

1-Methyl-7-(4-methyl-1-piperazinyl)-4-oxo-4H[1,3]-thiazeto[3',2'-1,2]pyrido[2,3-d]pyrimidine-3-carboxylic acid:
0.300 g of the compound obtained in the step (3) of Referential Example was suspended in 10 ml of dry DMF. A solution of 0.212 g of N-methylpiperazine as dissolved in 5 ml of dry DMF was dropwise added thereto at room temperature. After the addition, the solvent was removed by distillation under reduced pressure, and the residue was dissolved in chloroform. The insoluble substances were taken out by filtration and the solvent was removed by distillation under reduced pressure. The residue was recrystallized from ethanol to obtain 168 mg of the intended compound. m.p. 236 to 238°C (decomposition).

| Elementary Analysis (C₁₅H₁₇N₅O₃S): | | | |
|---|---|---|---|
| Calculated Value (%) | C: 51.86, | H: 4.93, | N: 20.16 |
| Measured Value (%) | C: 51.47, | H: 4.72, | N: 19.88 |

### Example 8:

7-(4-Acetyl-1-[piperazinyl)-1-methyl-4-oxo-4H[1,3] thiazeto[3',2'-1,2]pyrido[2,3-d]pyrimidine-3-carboxylic acid:
0.466 g of the compound obtained in the step (3) of Referential Example was suspended in 15 ml of dry DMF. A solution of 0.423 g of N-acetylpiperazine dissolved in 10 ml of dry DMF was dropwise added thereto at room temperature and stirred for 30 minutes. After the reaction, the solvent was removed by distillation under reduced pressure, and the crystal formed was washed with ethanol and dried under reduced pressure to obtain 0.39 g of the intended compound. m.p. 271 to 272°C (decomposition).

| Elementary Analysis (C₁₆H₁₇N₅O₄S): | | | |
|---|---|---|---|
| Calculated Value (%) | C: 51.19, | H: 4.56, | N: 18.66 |
| Measured Value (%) | C: 51.02, | H: 4.43, | N: 18.46 |

### Example 9:

1-Methyl-7-(1-piperazinyl)-4-oxo-4H[1,3]thiazeto[3',2'-1,2]-pyrido[2,3-d]pyrimidine-]-3-carboxylic acid:
257.5 ml of the compound obtained in Example 8 was suspended in 30 ml of 5 % hydrochloric acid and refluxed for 3.5 hours. The reaction mixture was left as such overnight. The crystal precipitated was taken out by filtration, washed with ethanol and dried under reduced pressure to obtain 57.7 mg of the intended compound. m.p. 300°C or higher.

| Elementary Analysis (C₁₄H₁₅N₅O₃S·HCl): | | | |
|---|---|---|---|
| Calculated Value (%): | C: 45.47, | H: 4.36, | N: 18.94 |
| Measured Value (%): | C: 45.22, | H: 4.44, | N: 18.80 |

I R ν ₘₐₓ cm⁻¹: 3420, 2810, 2405, 1705, 1620, 1455, 1430, 1355, 1325, 1025, 810.

In the same manner as Examples 1 to 9, the following compounds were obtained.

### Example 10:

Ethyl 7-(4-Acetyl-1-piperazinyl)-6-fluoro-1-methyl-4-oxo-4H[1,3]thiazeto[3,2-a]-1,8-naphthyridine-3-carboxylate:
m.p. 203 to 205°C

| Elementary Analysis (C₁₉H₂₁FN₄O₄S): | | | |
|---|---|---|---|
| Calculated Value (%) | C: 54.28, | H: 5.03, | N: 13.33 |
| Measured Value (%): | C: 54.10, | H: 5.04, | N: 13.17 |

### Example 11:

Ethyl 7-(4-Acetyl-1-piperazinyl)-1-ethyl-6-fluoro-4-oxo-4H[1,3]thiazeto[3,2-a]-1,8-naphthyridine-3-carboxylate:
N M R δ (CDCl₃ ): 1.12(3H, t), 1.37(3H, t), 2.12(3H, s), 3.40 ~4.00(8H, m), 4.30(2H, q), 5.50~6.00(2H, m), 7.90(1H, d)

### Example 12:

6-Fluoro-1-methyl-4-oxo-7-(1-piperazinyl)-4H[1,3]thiazeto[3,2-a]-1,8-naphthyridine-3-carboxylic acid hydrochloride:
m.p. 280°C (decomposition).

| Elementary Analysis (C₁₅H₁₅FN₄O₃S·HCl): | | | |
|---|---|---|---|
| Calculated Value (%) | C: 46.57, | H: 4.17, | N: 14.48 |
| Measured Value (%) | C: 46.68, | H: 4.10, | N: 14.28 |

### Example 13:

1-Ethyl-6-fluoro-7-(1-piperazinyl)-4-oxo-4H[1,3]thiazeto[3,2-a]-1,8-napthyridine-3-carboxylic acid:
m.p. 207 to 209°C.

| Elementary Analysis (C₁₆H₁₇FN₄O₃S·4H₂O): | | | |
|---|---|---|---|
| Calculated Value (%) | C: 44.03, | H: 5.77, | N: 12.84 |
| Measured Value (%) | C: 43.75, | H: 5.63, | N: 12.86 |

### Example 14:

6-Fluoro-1-methyl-7-(4-methyl-1-piperazinyl)-4-oxo-4H[1,3]thiazeto[3,2-a]-1,8-napthyridine-3-carboxylic acid:
m.p. 250 to 252°C (decomposition).

| Elementary Analysis (C₁₆H₁₇FN₄O₃S): | | | |
|---|---|---|---|
| Calculated Value (%) | C: 52.74, | H: 4.70, | N: 15.38 |
| Measured Value (%) | C: 52.81, | H: 4.72, | N: 14.91 |

### Example 15:

1-Ethyl-6-fluoro-7-(4-methyl-1-piperazinyl)-4-oxo-4H[1,3]thiazeto[3,2-a]-1,8-naphthyridine-3-carboxylic acid:
m.p. 237°C.

| Elementary Analysis (C₁₇H₁₉FN₄O₃S·1/2 H₂O): | | | |
|---|---|---|---|
| Calculated Value (%) | C: 52.70, | H: 5.20, | N: 14.46 |
| Measured Value (%) | C: 52.78, | H: 4,76, | N: 14.60 |

### Example 16:

Ethyl 7-(4-Ethoxycarbonyl-1-piperazinyl)-6-fluoro-4-oxo-4H[1,3]thiazeto[3,2-a]-1,8-naphthyridine-3-carboxylate:
m.p. 205 to 229°C (decomposition).

| Elementary Analysis (C₁₉H₂₁FN₄O₃S): | | | |
|---|---|---|---|
| Calculated Value (%) | C: 52.29, | H: 4.85, | N: 12.84 |
| Measured Value (%) | C: 51.94, | H: 4.92, | N: 12.68 |

### Example 17:

Ethyl 6-Fluoro-1-methyl-7-morpholino-4-oxo-4H[1,3] thiazeto[3,2-a]-1,8-naphthyridine-3-carboxylate:
m.p. 198 to 202°C (decomposition).

| Elementary Analysis (C₁₇H₁₈FN₃O₄S): | | | |
|---|---|---|---|
| Calculated Value (%) | C: 53.82, | H: 4.78, | N: 11.08 |
| Measured Value (%) | C: 53.64, | H: 4.69, | N: 10.92 |

### Example 18:

6-Fluoro-1-methyl-7-morpholino-4-oxo-4H[1,3]thiazeto-[3,2-a]-1,8-napthyridine-3-carboxylic acid:
m.p. 249°C (decomposition).

| Elementary Analysis (C₁₅H₁₄FN₃O₄S): | | | |
|---|---|---|---|
| Calculated Value (%) | C: 51.28, | H: 4.02, | N: 11.96 |
| Measured Value (%) | C: 51.30, | H: 4.02, | N: 11.68 |

### Example 19:

6-Fluoro-7-morpholino-4-oxo-4H[1,3]thiazeto[3,2-a]-1,8-naphthyridine-3-carboxylic acid:
m.p. 280 to 283°C (decomposition).

### Example 20:

6-Chloro-4-oxo-7-(1-piperazinyl)-4H[1,3]thiazeto[3,2-a]-1,8-naphthyridine-3-carboxylic acid hydrochloride:
m.p. 250°C (decomposition).

| Elementary Analysis (C₁₄H₁₃ClN₄O₃S·HCl) | | | |
|---|---|---|---|
| Calculated value (%) | C: 43.20, | H: 3.63, | N: 14.39 |
| Measured Value (%) | C: 43.12, | H: 3.39, | N: 14.23 |

### Example 21:

6-Chloro-1-methyl-4-oxo-7-(1-piperazinyl)-4H[1,3]thiazeto [3,2-a]-1,8-naphthyridine-3-carboxylic acid hydrochloride:
m.p. 280°C (decomposition).

| Elementary Analysis (C₁₅H₁₅ClN₄O₃S·HCl): | | | |
|---|---|---|---|
| Calculated Value (%) | C: 44.67, | H: 4.00, | N: 13.89 |
| Measured Value (%) | C: 44.69, | H: 3,82, | N: 13.72 |

### Example 22:

4-Chloro-7-(4-methyl-1-piperazinyl)-4-oxo-4H[1,3]thiazeto [3,2-a]-1,8-naphthyridine-3-carboxylic acid:
m.p. 240°C (decomposition).

| Elementary Analysis (C₁₅H₁₅ClN₄O₃S·H₂O): | | | |
|---|---|---|---|
| Calculated Value (%) | C: 46.82, | H: 4.45, | N: 14.56 |
| Measrued Value (%) | C: 46.80, | H: 4.34, | N: 14.45 |

### Example 23:

6-Chloro-7-(4-methyl-1-piperazinyl)-4-oxo-4H[1,3]-thiezeto[3,2-a]-1,8-naphthyridine-3-carboxylic acid ethanesulfonate:
m.p. 260 to 263°C.

| Elementary Analysis (C₁₅H₁₅ClN₄O₃S·C₂H₆O₃S·2H₂O): | | | |
|---|---|---|---|
| Calculated Value (%) | C: 39.80, | H: 4.91, | N: 10.92 |
| Measured Value (%) | C: 40.06, | H: 4.76, | N: 10.80 |

### Example 24:

6-Chloro-1-methyl-7-(4-methyl-1-piperazinyl)-4-oxo-4H-[1,3]thiazeto[3,2-a]-1,8-naphthyridine-3-carboxylic acid:
m.p. 252 to 254°C (decomposition).

| Elementary Analysis (C₁₆H₁₇ClN₄O₃S): | | | |
|---|---|---|---|
| Calculated Value (%) | C: 50.46, | H: 4.50, | N: 14.71 |
| Measured Value (%) | C: 50.36, | H: 4.50, | N: 14.58 |

### Example 25:

6-Chloro-1-methyl-7-(4-methyl-1-piperazinyl)-4-oxo-4H-[1,3]thiazeto[3,2-a]-1,8-naphthyridine-3-carboxylic acid ethanesulfonate:
m.p. 262 to 264°C.

| Elementary Analysis (C₁₆H₁₇ClN₄O₃S·C₂H₆O₃S·3/2 H₂O): | | | |
|---|---|---|---|
| Calculated Value (%) | C: 41.74, | H: 5.06, | N: 10.82 |
| Measured Value (%) | C: 41.80, | H: 5.18, | N: 10.71 |

### Example 26:

7-(4-Methyl-1-piperazinyl)-4-oxo-4H[1,3]thiazeto[3',2'-1,2] pyrido[2,3-d)pyrimidine-3-carboxylic acid:
m.p. 300°C or higher.

| Elementary Analysis (C₁₄H₁₅N₅O₃S): | | | |
|---|---|---|---|
| Calculated Value (%) | C: 50.44, | H: 4.54, | N: 20.52 |
| Measured Value (%) | C: 49.90, | H: 4.72, | N: 20,52 |

I R ν ₘₐₓ cm⁻¹: 3400, 1695, 1620, 1465, 970, 805.

### Example 27:

Ethyl 7-(4-Acetyl-1-piperazinyl)-6-chloro-4-oxo-4H[1,3] thiazeto[3,2-a]-1,8-naphthyridine-3-carboxylate:
N M R δ (CDCl₃): 1.33(3H, t), 2.11(3H. s) 3.30~3.90(8H,m), 4.29(2H, q), 5.38(2H. s), 8.32(1H, s)

### Example 28:

Ethyl 7-(4-Acetyl-1-piperazinyl)-6-chloro-1-methyl-4-oxo-4H[1,3]thiazeto[3,2-a]-1,8-naphthyridine-3-carboxylate:
N M R δ (CDCl₃): 1.35(3H, t), 2.12(3H, s) 2.13(3H, d), 3.20 ~3.90(8H,m), 4.30(2H, q) 5.94(1H, q), 8.32(1H, s)

### Example 29.

Ethyl 7-(4-acetyl-1-piperazinyl)-6-fluoro-1-phenyl-4-oxo-4H[1,3]thiazeto[3,2-a]1,8 naphthyridine-3-carboxylate. Mass analysis (C₂₄H₂₃FN₄O₄S), M⁺: 482.

### Example 30.

Ethyl 7-(4-acetyl-1-piperazinyl)-6-fluoro-1-(4-fluorophenyl)-4-oxo-4H[1,3]thiazeto[3,2-a]-1,8-naphthyridine-3-carboxylate. Mass analysis (C₂₄H₂₂F₂N₄O₄S), M⁺: 500.

### Example 31.

Ethyl 7-(4-acetyl-1-piperazinyl)-1-(3,4-difluorophenyl)-6-fluoro-4-oxo-4H[1,3]thiazeto[3,2-a]-1,8-naphthyridine-3-carboxylate. Mass analysis (C₂₄H₂₁F₃N₄O₄S), M⁺: 518.

### Example 32.

Ethyl 7-(4-acetyl-1-piperazinyl)-1-(2,4-difluorophenyl)-6-fluoro-4-oxo-4H[1,3]thiazeto[3,2-a]-1,8-naphthyridine--3-carboxylate. Mass analysis (C₂₄H₂₁F₃N₄O₄S), M⁺: 518.

### Example 33.

Ethyl 7-(4-acetyl-1-piperazinyl)-1-(3,5-difluorophenyl)-6-fluoro-4-oxo-4H[1,3]thiazeto[3,2-a]-1,8-naphthyridine-3-carboxylate. Mass analysis (C₂₄H₂₁F₃N₄O₄S), M⁺: 518.

### Example 34.

6-Fluoro-1-phenyl-7-piperazinyl-4-oxo-4H [1,3]thiazeto[3,2-a]-1,8-naphthyridine-3-carboxylic acid hydrochloride. M.p. 250°C (decompn.). Mass analysis (C₂₀H₁₇FN₄O₃S.HCl), M⁺: 448.

| Elementary analysis for C₂₀H₁₇FN₄O₃S.HCl.H₂O: | | | |
|---|---|---|---|
| Calcd: | C 51.45, | H 4.31, | N 12.00 |
| Found: | C 51.43, | H 4.10, | N 12.19 |

### Example 35.

6-fluoro-1-(4-fluorophenyl)-7-piperazinyl-4-oxo-4H[1,3]thaizeto[3,2-a]-1,8-naphthyridine-3-carboxylic acid. M.p. 260°C (decompn.). Mass analysis (C₂₀H₁₆F₂N₄O₃S), M⁺ : 430.

| Elementary analysis for C₂₀H₁₆F₂N₄O₃S·2H₂O | | | |
|---|---|---|---|
| Calcd (%) | C 51.50, | H 4.32, | N 12.01 |
| Found (%) | C 49.91, | H 4.19, | N 12.63 |

### Example 36.

1-(2,4-difluorophenyl)-6-fluoro-7-piperazinyl-4-oxo-4H[1,3]thaizeto[3,2-a]-1,8-naphthyridine-3-carboxylic acid. M.p. 230°C (decompn). Mass analysis (C₂₀H₁₅F₃N₄O₃S), M⁺ : 448. Elementary analysis for C₂₀H₁₅S₃N₄O₃S.H₂O Calcd (%) C 51.50, H 3.67, N 12.01; Found (%) C 51.21, H 3.69 N 11.54

### Example 37.

1-(3,4-difluorophenyl)-6-fluoro-7-piperazinyl-4-oxo-4H[1,3]thiazeto[3,2-a]-1,8-naphthyridine-3-carboxylic acid. Mass analysis (C₂₀H₁₅F₃N₄O₃S), M⁺: 448.

### Example 38.

1-(3,5-difluorophenyl)-6-fluoro-7-piperazinyl-4-oxo-4H[1,3]thiazeto[3,2-a]-1,8-naphthyridine-3-carboxylic acid. Mass analysis (C₂₀H₁₅F₃N₄O₃S), M⁺: 448.

### Example 39.

6-fluoro-7-(4-methyl-1-piperazinyl)-1-phenyl-4-oxo-4H[1,3]thiazeto[3,2-a]-1,8-naphthyridine-3-carboxylic acid. M.p. 270°C (decompn.). Mass analysis (C₂₁H₁₉FN₄O₃S), M⁺ : 426.

### Example 40.

6-fluoro-1-(4-fluorophenyl)-7-(4-methyl-1-piperazinyl)-4-oxo-4H[1,3]thiazeto[3,2-a]-1,8-naphthyridine- 3-carboxylic acid. M.p. 270°C (decompn.). Mass analysis (C₂₁H₁₈F₂N₄O₃S), M⁺ : 444.

| Elementary analysis for C₂₁H₁₈F₂N₄O₃S.H₂O | | | |
|---|---|---|---|
| Calcd (%) | C 54.54, | H 4.36, | N 12.11 |
| Found (%) | C 54.88, | H 4.51, | N 11.37 |

### Example 41.

1-(3,4-difluorophenyl)-6-fluoro-7-(4-methyl-1-piperazinyl)-4-oxo-4H[1,3]thiazeto[3,2-a]-1,8-naphthyridine-3-carboxylic acid. Mass analysis (C₂₁H₁₉F₃N₄O₃S), M⁺ : 502.

### Example 42.

1-(2,4-difluorophenyl)-6-fluoro-7-(4-methyl -1-piperazinyl)-4-oxo-4H[1,3]thiazeto[3,2-a]-1,8-naphthyridine-3-carboxylic acid. Mass analysis (C₂₁H₁₉F₃N₄O₃S), M⁺ : 502.

### Example 43.

1-(3,5-difluorophenyl)-6-fluoro-7-(4-methyl-1-piperazinyl)-4-oxo-4H[1,3]thiazeto[3,2-a]-1,8-naphthyridine-3-carboxylic acid. Mass analysis (C₂₁H₁₉F₃N₄O₃S), M⁺ : 502.

### Example 44.

Ethyl 6-fluoro-7-(4-methyl-1-piperazinyl)-1-phenyl-4-oxo-4H[1,3]thiazeto[3,2-a]-1,8-naphthyridine-3-carboxylate.

A mixture of 200 mg of the compound obtained in Example 39, 64 mg of potassium carbonate, 74 mg of ethyl iodide and 6ml of N,N-dimethylformamide were stirred at room temperature for 16 hours. 60 ml of water was added to the reaction mixture and than extracted with chloroform. The chloroform layer was washed with water, dried and then concentrated and the resultant residue was purified on silica gel chromatography to obtain 120 mg of crystals. M.p. 197 - 198°C. Mass analysis (C₂₃H₂₃FN₄O₃S), M⁺ : 454.

Elementary anaylsis for C₂₃H₂₃FN₄O₃S.Y₂H₂O Calcd (%): C 59.60, H 5.21, N 12.09; Found (%) C 59.69, H 5.15, N 11.99
The following compounds were obtained in the same manner as in Example 44.

### Example 45.

Ethyl 6-fluoro-1-(4-fluorophenyl)-7-(4-methyl-1-piperazinyl)-4-oxo-4H[1,3]thiazeto[3,2-a]-1,8-naphthyridine-3-carboxylate. Mass analysis (C₂₃H₂₂F₂N₄O₃S), M⁺ : 472.

### Example 46.

Ethyl 1-(3,4-difluorophenyl)-6-fluoro-7-(4-methyl-1-piperazinyl)-4-oxo-4H[1,3]thiazeto[3,2-a]-1,8-naphthyridine-3-carboxylate. Mass analysis (C₂₃H₂₁F₃N₄O₃S), M⁺ : 490.

### Example 47.

Ethyl 1-(2,4-difluorophenyl)-6-fluoro-7-(4-methyl-1-piperazinyl) -4-oxo-4H[1,3]thiazeto[3,2-a]-1,8-naphthyridine-3-carboxylate. Mass analysis (C₂₃H₂₁F₃N₄O₃S), M⁺ : 490.

### Example 48.

Ethyl 1-(3,5-difluorophenyl)-6-fluoro-7-(4-methyl-1-piperazinyl)-4-oxo-4H[1,3]thiazeto[3,2-a]-1,8-naphthyridine-3-carboxylate. Mass analysis (C₂₃H₂₁F₃N₄O₃S), M⁺ : 490.

### Example 49.

1-(2,4-difluorophenyl)-6-fluoro-7-(4-(5-methyl-2-oxo-1,3-dioxolen-4-yl)methyl-1-piperazinyl)-4-oxo-4H[1,3]thiazeto[3,2-a]-1,8-naphthyridine-3-carboxylic acid.

449 mg or the compound obtained in Example 36. 120 mg of potassium hydrogen carbonate were suspended in 5 ml of N,N-dimethylformamide, to which 232 mg of 4-bromomethyl-5-methyl-1,3-dioxolen-2-one was dropped under ice cooling and stirred for 3 hours. After the reaction was over the solvent was distilled off at 60°C under a reduced pressure. Iced water was added to the resultant residue and insoluble matters were collected by filtration, washed with water and then dried in air. Crude crystals were recrystallized from a chloroform-methanol mixed solution to obtain an aimed product. M.p. 215°C (decompn) Mass analysis (C₂₅H₁₉F₃N₄O₃S), M⁺ : 560.

Elementary analysis for C₂₅H₁₉F₃N₄O₆S. Calcd (%): C 53.57, H 3.42, N 10.00; Found (%) C 52.86, H 3.53, N 9.78
The following compounds were obtained in the same manner as in Example 49.

### Example 50.

6-fluoro-7-(4-(5-methyl-2-oxo-1,3-dioxolen-4-yl)methyl-1-piperazinyl)-1-phenyl-4-oxo-4H[1,3]thiazeto [3,2-a]-1,8-naphthyridine-3-carboxylic acid. Mass analysis (C₂₅H₂₁FN₄O₆S), M⁺: 524.

### Example 51.

6-fluoro-1-(4-fluorophenyl)-7-(4-(5-methyl-2-oxo-1,3-dioxolen-4-yl)methyl-1-piperazinyl)-1-phenyl-4-oxo-4H[1,3]thiazeto[3,2-a]-1,8-naphthyridine-3-carboxylic acid. Mass analysis (C₂₅H₂₀F₂N₄O₆S), M⁺: 542.

### Example 52.

1-(3,4-difluorophenyl)-6-fluoro-7-(4-(5-methyl-2-oxo-1,3-dioxolen-4-yl)methyl-1-piperazinyl)-4-oxo-4H[1,3]thiazeto[3,2-a]-1,8-naphthyridine-3-carboxylic acid. Mass analysis (C₂₅H₁₉F₃N₄O₃S), M⁺ : 560.

### Example 53.

1-(3,5-difluorophenyl)-6-fluoro-7-(4-(5-methyl-2-oxo-1,3-dioxolen-4-yl)methyl-1-piperazinyl)-4-oxo-4H[1,3]thiazeto[3,2-a]-1,8-naphthyridine-3-carboxylic acid. Mass analysis (C₂₅H₁₉F₃N₄O₃S), M⁺ : 560.

### Example 54.

Ethyl 6-fluoro-1-(4-fluorophenyl)-7-piperazinyl-4-oxo-4H[1,3]thiazeto[3,2-a]-1,8-naphthyridine-3-carboxylate.

430 mg of the compound obtained in Example 35 was dissolved in 15 ml of 95 % formic acid, to which 5 ml of anhydrous acidic acid was dropped under ice cooling and stirring. After stirring the reaction mixture at room temperature for 4 hours, it was poured into iced water and deposited crystals were collected by filtration, washed with water and ethanol and then dried. Then, a mixture of the resultant crystals, 138 mg of potassium carbonate, 156 mg or methyl iodide and 10 ml of N,N-dimethylformamide was stirred at room temperature over one night. 100 ml of water was added to the reaction solution and extracted with chloroform. The chloroform layer was washed with water, dried and then concentrated. Successively, 12 ml of ethanol and 0.24 ml of concentrated sulfuric acid were added to the resultant residue and then refluxed under heating et 80 - 90°C For 4.5 hours. After condensating the reaction solution under a reduced pressure, 10 ml of water was added, neutralized with sodium hydrogen carbonate and then extracted with chloroform. The chloroform layer was washed with water, dried and then concentrated under a reduced pressure. The residue was purified on silica gel chromatography to obtain the above-captioned compound.
Mass analysis (C₂₂H₂₀F₂N₄O₃S), M⁺ : 458.

The following compounds were obtained in the same manner as in Example 54.

### Example 55.

Ethyl 6-Fluoro-1-phenyl-7-piperazinyl-4-oxo-4H[1,3]thiazeto[3,2-a]-1,8-naphthyridine-3-carboxylate. Mass analysis (C₂₂H₂₁FN₄O₃S), M⁺ : 440.

### Example 56.

Ethyl 1-(3,4-difluorophenyl)-6-fluoro-7-piperazinyl-4-oxo-4H[1,3]thiazeto[3,2-a]-1,8-naphthyridine-3-carboxylate. Mass analysis (C₂₂H₁₉F₃N₄O₃S), M⁺ : 476.

### Example 57.

Ethyl 1-(2,4-difluorophenyl)-6-fluoro-7-piperazinyl-4-oxo-4H[1,3]thiazeto[3,2-a]-1,8-naphthyridine-3-carboxylate. Mass analysis (C₂₂H₁₉F₃N₄O₃S), M⁺ : 476.

### Example 58.

Ethyl 1-(3,5-difluorophenyl)-6-fluoro-7-piperazinyl-4-oxo-4H[1,3]thiazeto[3,2-a]-1,8-naphthyridine-3-carboxylate. Mass analysis (C₂₂H₁₉F₃N₄O₃S), M⁺ : 476.

### Experimental Example:

Typical examples of the compounds of the present invention were subjected to the following tests so as to prove the useful pharmaceutical effects of them.

### 1. Measurement of Minimal Growth Inhibitory Concentration (MIC):

### Test Method:

MIC was measured by agar plate dilution assay method in accordance with the standard by Japan Chemical Therapeutics Association (refer to Journal of Japan Chemical Therapeutics Association, 29 (1), 76 to 79 (1981)).

Briefly, a bacteria suspension as incubated in a bouillon medium for measurement of sensitivity, at 37°C for 18 hours was diluted to 10⁶ CFU/ml with the same medium. This was inoculated on an agar medium for measurement of sensitivity, which contained a chemical agent to be tested, with a microplanter and incubated at 37°C for 18 horus and then MIC was measured. As a control agent was used enoxacin. The results obtained were shown in Table 1 below. As is obvious therefrom, the compounds of the present invention had an extremely strong bactericidal activity against Pseudomonas aeruginosa and other gram-positive bacteria and gram-negative bacteria.

### 2. Remedial Effect against Mouse Infectious Diseases:

### Test Method:

E. coli KC-14 and P. aeruginosa E-2 each were suspended in 4 % mucin, and 0.23 ml of each of the resulting suspensions was inoculated into the abdominal cavity of ddy male mice (weight: about 20 g, 4-week age, 10 mice for one group). The amount of the bacteria inoculated was 5.1 x 10⁴ CFU/mouse for E. coli KC-14 and 7.5 x 10⁴ CFU/mouse for P. aeruginosa E-2. Two hours after the inoculation of the bacteria, the chemical agent to be tested was perorally administered once, and the number of the living animals after one week was counted. The results obtained were compared with those using the control enoxacin. The results were shown in Table 1 below.

The compounds of the present invention had a strong remedial effect to mouse infectious diseases. In particular, the activity against P. aeruginosa E-2 was higher than enoxacin which is said strongly effective against the said bacteria.

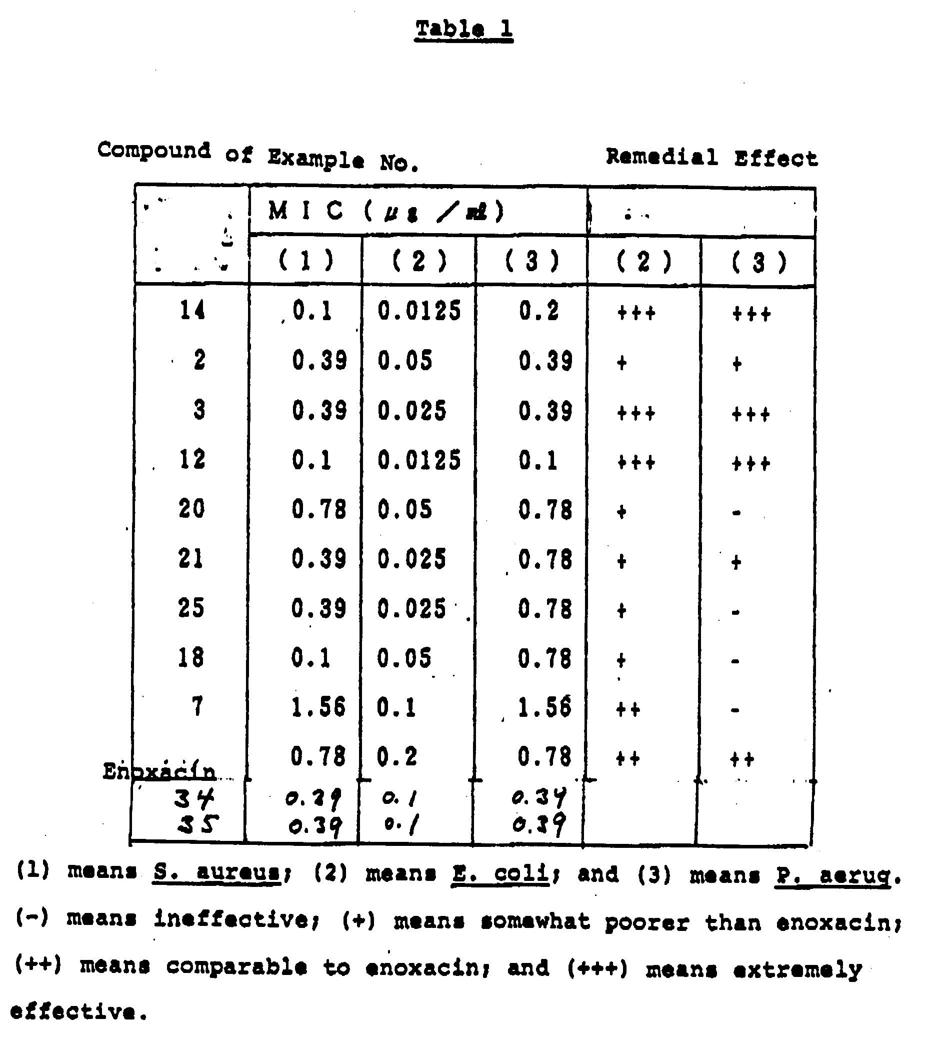

### (Advantage of the Invention)

The compounds of the present invention are effective not only to P. aeruginosa but also to various gram-positive and gram-negative bacteria even when they are used in a far smaller amount than any other known bactericides. The compounds of the present invention have sufficient bactericidal spectra in a broad range.

In addition, the toxicity of the compounds of the present invention is extremely low. Accordingly, the compounds of the present invention can safely be applied to mammals inclusive of human for remedy of systemic infectious diseases as well as local infectious diseases such as urinary tract infectious diseases or chologenic tract infectious diseases.

## Claims

1. Thiazetidine derivatives of the following general formula (I) and pharmaceutically acceptable salts thereof. 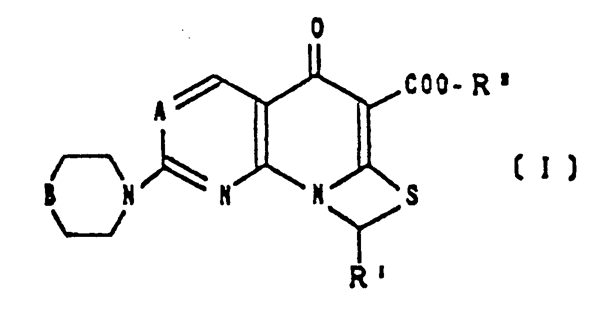 in which R¹ represents hydrogen, a linear or branched lower alkyl group having from 1 to 6 carbon atoms or phenyl, 4-fluorophenyl, 2,4-difluorophenyl, 3,4-difluorophenyl or 3,5-difluorophenyl; R² represents hydrogen or linear or branched lower alkyl group having from 1 to 6 carbon atoms; A represents N or CX, where X represents a halogen atom; and B represents O or NR³, where R³ represents a hydrogen atom, a linear or branched lower alkyl group having from 1 to 6 carbon atoms, a linear or branched acyl group having from 1 to 6 carbon atoms, trifluoroacetyl or a linear or branched alkoxycarbonyl group having from 2 to 5 carbon atoms or (5-methyl-2-oxo-1,3-dioxolen-4-yl)methyl.

## Patentansprüche

1. Thiazetidin-Derivate der folgenden allgemeinen Formel (I) und deren pharmazeutisch akzeptable Salze 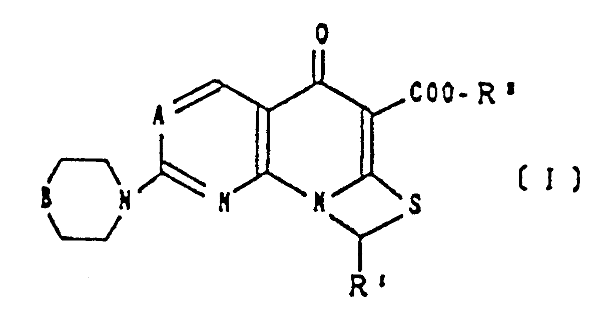 worin R1 Wasserstoff, eine lineare oder verzweigte niedrige Alkylgruppe mit 1 bis 6 Kohlenstoffatomen, eine Phenyl-, 4-Fluorphenyl-, 2,4-Difluorphenyl-, 3,4-Difluorphenyl- oder 3,5-Difluorphenylgruppe ist; R2 Wasserstoff oder eine lineare oder verzweigte niedrige Alkylgruppe mit 1 bis 6 Kohlenstoffatomen ist; A gleich N oder CX ist, wobei X ein Halogenatom darstellt; und B gleich O oder NR³ ist, wobei R³ ein Wasserstoffatom, eine lineare oder verzweigte niedrige Alkylgruppe mit 1 bis 6 Kohlenstoffatomen, eine lineare oder verzweigte Acylgruppe mit 1 bis 6 Kohlenstoffatomen, eine Trifluoracetyl- oder eine lineare oder verzweigte Alkoxycarbonylgruppe mit 2 bis 5 Kohlenstoffatomen oder ein (5-Methyl-2-Oxo-1,3-Dioxolen-4-yl)Methyl ist.

## Revendications

1. Dérivés de la thiazétidine répondant à la formule générale (I) suivante, et leurs sels pharmaceutiquement acceptables 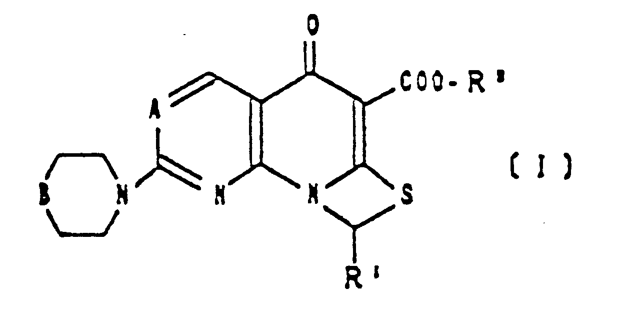 dans laquelle R¹ représente un atome d'hydrogène, un groupe alkyle inférieur linéaire ou ramifié en C₁ à C₆ ou un groupe phényle, 4-fluorophényle, 2,4-difluorophényle, 3,4-difluorophényle ou 3,5-difluorophényle; R² représente un atome d'hydrogène ou un groupe alkyle inférieur linéaire ou ramifié en C₁ à C₆, A représente N ou CX, où X représente un atome d'halogène; et B représente O ou NR³, où R³ représente un atome d'hydrogène, un groupe alkyle inférieur linéaire ou ramifié en C₁ à C₆, un groupe acyle linéaire ou ramifié en C₁ à C₆, un groupe trifluoracétyle ou un groupe alcoxycarbonyle linéaire ou ramifié en C₂ à C₅, ou un groupe (5-méthyl-2-oxo-1,3-dioxolén-4-yl)méthyle.
